# EUROPEAN PATENT SPECIFICATION

(11) **EP 3 515 377 B1**
(45) Date of publication and mention of the grant of the patent: **16.12.2020**
(21) Application number: 17777726.5
(22) Date of filing: 19.09.2017
(51) Int. Cl.: A61F 5/02

(54) **SPINAL ORTHOSIS**
WIRBELSÄULENORTHESE
ORTHÈSE DU TRONC

(30) Priority: 20.09.2016 US 201615270155
(43) Date of publication of application: 31.07.2019
(73) Proprietor: Ossur Iceland EHF, 110 Reykjavik (IS)
(72) Inventor: KLUTTS, Zachariah J., Foothill Ranch, CA 92610 (US)
(74) Representative: Delorme, Nicolas
(86) International application number: PCT/US2017/052143
(87) International publication number: WO 2018/057478

(56) References cited:
- WO-A1-2007/003148
- WO-A1-2016/138215
- US-A1- 2014 081 189

## Description

### FIELD OF THE DISCLOSURE

The disclosure relates generally to orthopedic devices, and more specifically to a spinal orthosis.

### BACKGROUND

A spinal orthosis or lumbar support is an orthopedic device designed for pain relief, protecting injured ligaments or muscles and post-surgical immobilization. A spinal orthosis is arranged to relieve pressure over the spinous processes while applying an even pressure to the paraspinal musculature to ensure comfortable and effective healing. Typical indications for spinal orthoses include spinal stenosis, herniated discs, post-surgical stabilization, stable and non-displaced spinal fractures, spondylolithesis, spondylolysis, and degenerative spinal pathologies.

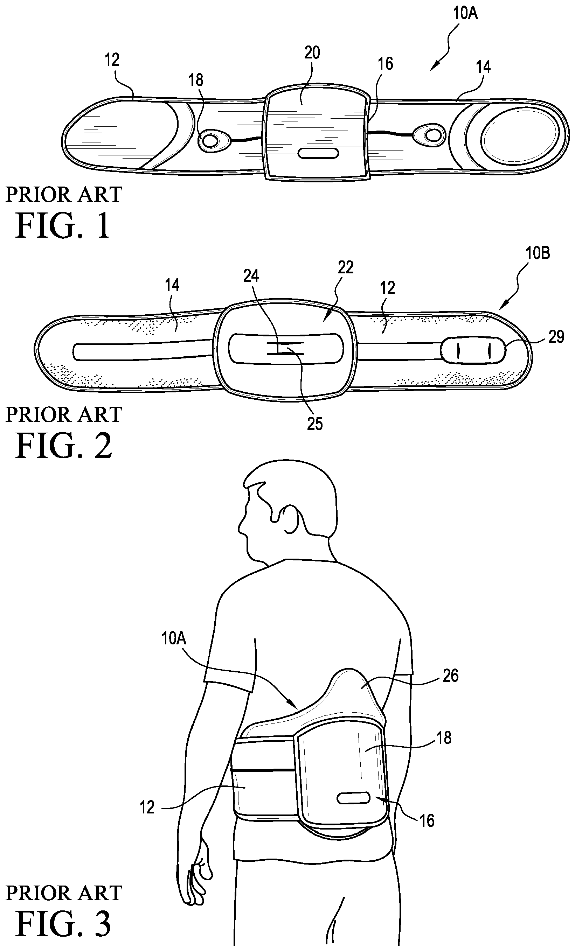

In a known spinal orthosis in Figs. 1-3, such as the exemplary spinal orthosis described in U.S. patent 8,172,779, granted on May 8, 2012 **,** the spinal orthosis has outer and inner side configurations 10A, 10B, with the inner side arranged to be adjacent the user's back. The orthosis has first and second belt members 12, 14, and a compression or closure system 16 adapted to exert pressure onto the lumbar region of a user's back. The compression or closure system 16 includes tightening elements or drawstrings 18 that permit the user to adjust pressure over the back and a cover 20 extending over the compression system 16.

A flexible or semi-rigid back plate 22 extends over at least part of the compression system 16, and is arranged to be adjacent the back of the user. The back plate 22 includes a posterior attachment system 24 for a rigid posterior panel 26, including a single hook and loop system connected at a single attachment point or flap 25 centered on the back plate 22. An anterior panel may be attached to the spinal orthosis at an anterior attachment system on one of the belts.

A requirement in a spinal orthoses is that they immobilize, at least in part, the torso, and stabilize the back. A factor in achieving this requirement is that the spinal orthosis is properly sized according to the anatomy of the user, and allows the user to effectively position and fasten the spinal orthosis.
A spinal orthosis is disclosed in US 2014/0081189.

It is desired to provide only a few spinal orthosis sizes. There is a need for a spinal orthosis that permits resizing of belt lengths to offer a "one-size-fits-all" spinal orthosis, including means, if necessary, to facilitate resizing of the spinal orthosis as a user undergoes size changes during rehabilitation.

### SUMMARY

The present invention relates to a spinal orthosis as set out in the claims. According to the embodiments described herein, a spinal orthosis is arranged for creating circumferential compression for a user, particularly in the lumbar region of the spine. The spinal orthosis enables saggital and/or coronal control, while offering a superiorly comfortable spinal orthosis. Indications for the spinal orthosis may include spinal stenosis, herniated disc, and degenerative spinal pathologies. The spinal orthosis may be combined with rigid panels for post-surgical stabilization; stable, non-displaced spinal fractures; spondylolisthesis; spondylolysis; spinal stenosis; herniated disc; and degenerative spinal pathologies.

Various embodiments are arranged with significant improvement over known spinal orthoses in donning and fitting processes. The donning and fitting processes may be made without measurements, and catered to anatomy and changing anatomy of a given user. The donning and fitting is arranged so it can be done on the actual user and thereby particularly customized for optimal fit and performance. No trimming is required for donning and fitting to a user, and no complicated fasteners or buckles are required for donning and fitting to a user. The length of the belt members can be increased, such as with belt extenders, or reduced, by folding, from an initial length, and such initial length can always be preserved for further adjustment of an actual length (such as including belt extenders or being folded and attached) of the belt member in the context of wearing the belt members.

The embodiments are arranged with flexible and breathable materials having improved performance over known spinal orthoses, including ventilation features offering optimal breathability in strategic locations. The spinal orthosis is arranged to accommodate many sizes by having means for expanding belt member lengths. The embodiments possess streamlined features which reduce weight, size and bulk over known spinal orthoses.

In the broadest aspect, the present invention relates to a spinal orthosis as set out in claim 1.

The first front closure may include a locking element defined on a first side and arranged to engage a corresponding slot defined on a first side by the second front closure for securing the first and second belt members to one another. The first front closure defines a pocket along a first side, and configured and dimensioned for inserting at least a finger thereinto for locating a second end of the first front closure relative to the second end of the second belt member. The front closures may define first and second clamping sections arranged to removably secure to opposed sides of the foldable portions of the belt members.

The second ends of the first and second belt members may each include fasteners extending from an outer side of the spinal orthosis for securing to a surface of the first and second belt members on the outer side of the spinal orthosis. For example, an entirety or substantial entirety (such as areas with the exception of the fasteners or ventilation features) of the belt members may be formed from hook receivable material. At least one surface of the belt members, such as either an outer surface or an inner surface opposite the outer surface and intended to be adjacent and face the body of the user, may define hook receivable material. The fasteners may be hook material that can engage along the length between the fasteners and the rear panel of the belt members themselves to allow for significant sizing of the belt members beyond predetermined settings of a small group of selections, as in the prior art.

The belt members are preferably of low profile in height and thickness. For example, the first and second belt members have a thickness in the range of 1 to 5 mm, and more preferably in the range of 1.5 to 2.5 mm. The belt members may be devoid of padding or spacer material, as in the prior art, and rather rely on both surfaces being formed by hook-receivable material with a substantially thin thickness, identified as being within the range noted above. The belt members may include ventilation features defined by the belt members themselves, such that the belt members are continuously constructed from the same material between the first and second ends. The ventilation features may include perforations of a defined shape and size, and may include a region thereabout having a reduced thickness from the remainder of the thickness of the belt members. The belt members may include reduced thickness regions without the perforations, to facilitate bending of the belt members and contribute to overall comfort and compliance of the belt members to the user.

The rear panel preferably includes a closure system having at least one tensioning element with a handle. The at least one tensioning element extends from the closure system and the handle is removably securable to a first surface of the first front closure. A rigid posterior plate having a connector may removably secure to an inner side of the rear panel to relieve pressure over spinous processes while applying an even pressure to paraspinal musculature to ensure comfortable and effective healing. Similarly, a rigid anterior plate may be secured to the inner side of the belt members at the anterior side of the spinal orthosis. These rigid plates may be added or removed depending on the motion restriction and compression desired during a rehabilitation period.

In an embodiment, the rear panel includes a stretchable cover extending from the first and second sides. The cover is arranged for stretching or retracting over a variable distance according to adjustment of the closure system. The first and second belt members may be formed from a substantially non-stretchable material such that pulling the second ends of the first and second belt members causes the variable distance to increase, and releasing the second ends causes the cover to retract to a predetermined distance.

The rear panel includes a closure system and a first end of an elongate tensioning element engages the closure system, and a handle secures to a second end of the tensioning element. The first belt member forms a channel including a first opening proximate the closure system and a second opening. The channel is formed between first and second surfaces of the first belt member such that the tensioning element enters the channel at the first opening and exits by the second opening such that the second end of the tensioning element extends beyond the second opening.

The spinal orthosis may be provided as a kit including a rear panel having first and second sides, a first belt member having a first end secured to a first side of the rear panel and a resizable second end, and a second belt member having a first end secured to a second side of the rear panel and a resizable second end. A sizing device has first and second positioning elements for establishing a clearance defined by a predetermined distance between the second ends of the first and second belt members. The predetermined distance allows for same front closures to be used generally regardless of the length of a user's waist, such that the front closures do not require any trimming.

In an embodiment, the first and second positioning elements are first and second rods spaced apart by a cord having a length extending the predetermined distance between the first and second rods. In another embodiment, the sizing device is a board having a main portion and the first and second positioning elements extend from the main portion. The first and second positioning elements are spaced apart by a center section with a width forming the predetermined distance. First and second grooves are formed by the board and separate the first and second positioning elements, respectively, from the center section. The kit may include first and second belt expanders arranged for securing to the second end of the first and second belt members. The first and second belt expanders have first and second ends each bearing fasteners for securing to the first and second belt members.

Useful in connection with the present invention is a method for resizing a spinal orthosis comprises the steps of using a sizing device having first and second positioning elements for establishing a predetermined distance between the second ends of the first and second belt members. The method includes extending a portion of the second end of the first belt member over the first positioning element to determine a resized length of the first belt member between the rear panel and the first positioning element. The second end of the first belt member is secured over a peripheral surface of the first belt member to retain the resized length of the first belt member and form a foldable portion of the first belt member. The sizing device is removed after the resized length is established. A first front closure is secured to the foldable portion of the first belt member. A portion of the second end of the second belt member is extended over the second positioning element to determine a resized length of the second belt member between the rear panel and the second positioning element.

These and other features, aspects, and advantages of the present disclosure will become better understood regarding the following description, appended claims, and accompanying drawings.

### BRIEF DESCRIPTION OF THE DRAWINGS

Fig. 1 shows an outer side of a prior art spinal orthosis.
Fig. 2 shows an inner side of the prior art spinal orthosis of Fig. 1.
Fig. 3 is a perspective view of the prior art spinal orthosis of Fig. 1 having a posterior panel and placed on a user.
Fig. 4 is a perspective view of an example of a spinal orthosis.
Fig. 5 is a detail view of a ring of a front panel in the example of Fig. 4.
Fig. 6 is a detail view of a belt member extending through the ring of Fig. 5 and securing to itself.
Fig. 7A is a plan view of another example of a spinal orthosis.
Fig. 7B is a plan view of another example of a spinal orthosis.
Fig. 7C is a detail view taken from VIIC in Fig. 7B.
Fig. 7D is a detail view taken from VIID in Fig. 7B.
Fig. 7E is a belt expander for securing to the spinal orthosis of Fig. 7A.
Fig. 8 is a perspective plan view of various detachable components in the spinal orthosis of Fig. 7A.
Fig. 9A is a perspective view of a front closure for use in the spinal orthosis of Fig. 7A.
Figs. 9B and 9C are plan views showing inner and outer sides of a front closure for the spinal orthosis examples.
Fig. 9D is a schematic view showing attachment of the front closure in Figs. 9B and 9C to a spinal orthosis.
Fig. 9E is a schematic detail view showing the front closure in Figs. 9B and 9C attached to the spinal orthosis.
Fig. 10A is a plan view of a posterior panel for use with the spinal orthosis embodiments.
Fig. 10B is a plan view of the posterior panel of Fig. 10A including a cover.
Fig. 10C is a plan view of an anterior panel for use with the spinal orthosis embodiments.
Fig. 10D is a plan view of the anterior panel of Fig. 10C including a cover.
Fig. 10E is a plan view of another embodiment of a posterior panel for use with the spinal orthosis embodiments.
Fig. 10F is a plan view of the posterior panel of Fig. 10E including a cover.
Fig. 10G is a plan view of a lateral panel for use with the spinal orthosis embodiments.
Fig. 10H is a plan view of the lateral panel of Fig. 10G including a cover.
Fig. 11 is a schematic view of securing front closures to one another in the spinal orthosis of Fig. 7A.
Fig. 12A is a schematic view of tensioning elements in the spinal orthosis of Fig. 7A.
Fig. 12B is a schematic view of securing the tensioning elements to the front closures in the spinal orthosis of Fig. 7A.
Fig. 13 is a schematic view showing a method and kit for resizing the spinal orthosis of Fig. 7A by pulling belt members away from a sizing device.
Fig. 14 is a schematic view showing the method of Fig. 13 wherein the belt members are tensioned.
Fig. 15 is a plan view of an example of a sizing device.
Fig. 16 is a schematic view of a user using the sizing device of Fig. 15.
Fig. 17 is a plan view of the spinal orthosis of Fig. 7A including channels for a tensioning element.
Fig. 18 is a detail view of a first channel taken along detail XVI in Fig. 17.
Fig. 19 is a detail view of a second channel taken along detail XVII in Fig. 17.
Fig. 20 is a cross-sectional schematic view of the belt member in Fig. 19 including the second channel.
Figs. 21A and 21B are plan views of outside and inside portions of a closure embodiment in an open configuration for a first belt member.
Fig. 21C is a plan view of an outside portion of a closure embodiment of Fig. 21A in an open configuration for a second belt member.
Figs. 22A and 22B are plan views of the closure embodiment of Figs. 21A and 21B in a closed configuration.
Fig. 23 is an exploded view of a pull tab embodiment.
Fig. 24 is a plan view of the pull tab embodiment of Fig. 23.
Fig. 25 is a schematic view of the pull tab of Fig. 23 with a cable retainer.

The drawing figures are not drawn to scale, but instead are drawn to provide a better understanding of the components, and are not intended to be limiting in scope, but to provide exemplary illustrations.

### DETAILED DESCRIPTION OF VARIOUS EMBODIMENTS

A better understanding of different embodiments of the disclosure and background examples may be had from the following description read with the accompanying drawings in which like reference characters refer to like elements.

While the disclosure is susceptible to various modifications and alternative constructions, certain illustrative embodiments are in the drawings and described below. It should be understood, however, there is no intention to limit the disclosure to the specific embodiments disclosed, but on the contrary, the disclosure covers all modifications, alternative constructions, combinations, and equivalents falling within the scope of the invention as set out in the claims.

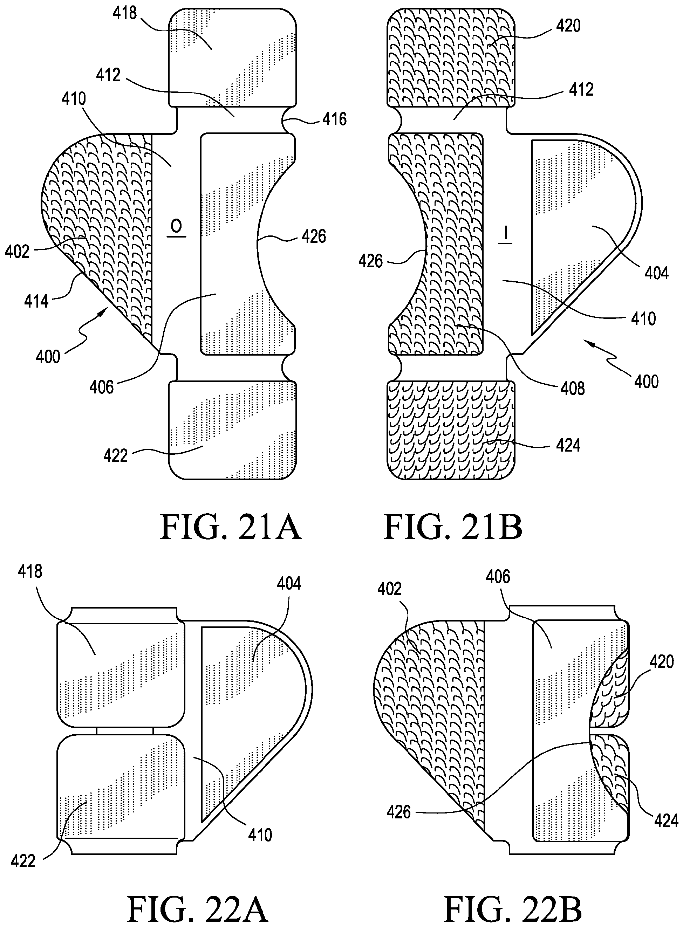

It will be understood that, unless a term is expressly defined in this disclosure to possess a described meaning, there is no intent to limit the meaning of such term, either expressly or indirectly, beyond its plain or ordinary meaning
Moreover, it will be understood that only the front closures disclosed in Figs. 21A-22B fall under the scope of protection as set out in the claims.

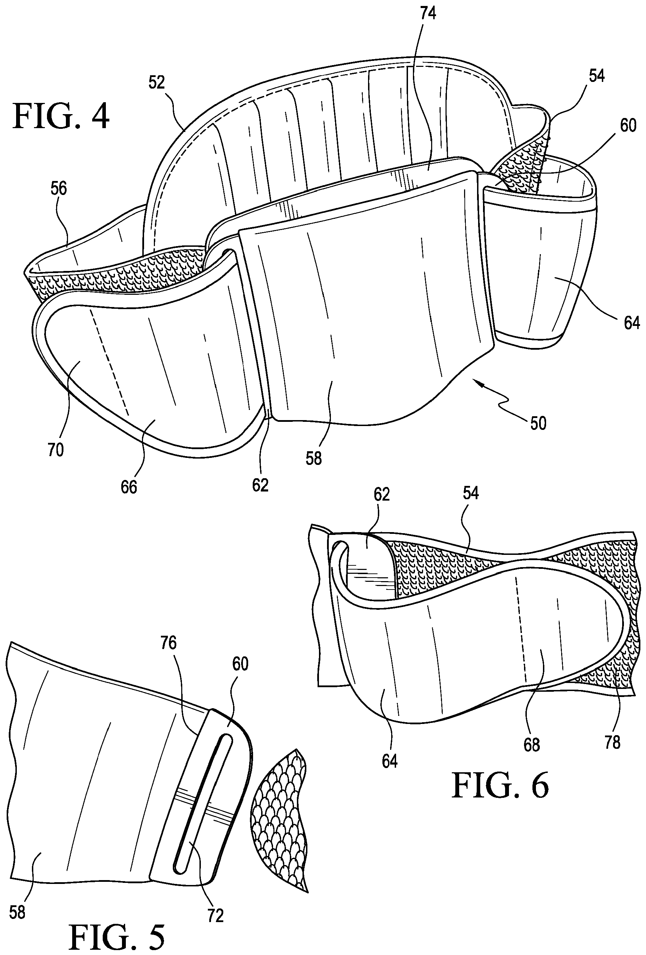

Figs. 4-6 illustrate an example of a spinal orthosis 50 having a posterior panel 52 and first and second belt members 54, 56 extending therefrom. An anterior panel 58 includes first and second rings 60, 62 having apertures 72 through which the first and second belt members 54, 56 extend. The first and second belt members 54, 56 include first and second folded parts 64, 66 that extend over the first and second rings 60, 62, in which end portions 68, 70 secure to outer surfaces 78 of the first and second belt members 54, 56. The first and second rings 60, 62 flexibly extend from the anterior panel 58 along flexible joints 76 to permit adjustment of the first and second belt members 54, 56 relative to the anatomy of the user. A rigid anterior plate 74 may be secured to the anterior panel 58.

The spinal orthosis 50 may include a compression or closure system (not shown) proximate the posterior panel as taught in U.S. patent 8,172,779 or U.S. patent application publication 2014/0207041, published on July 24, 2014 . The spinal orthosis 50 may be provided without the posterior panel 52, and may rather comprise only a compression system at the posterior side such that the spinal orthosis 50 forms a continuous circumference with the compression system, the first and second belt members 54, 56, and the anterior panel 58, as generally shown in Fig. 4.

The anterior panel 58 may be flexible, and can be either stretchable or non-stretchable. In the depicted example, the anterior panel 58 is preferably non-stretchable so the first and second belt members 54, 56 may be tensioned over the first and second rings 60, 62, as they are folded over the first and second rings 60, 62 and secured to the outer surface 78 of the first and second belt members 54, 56.

As depicted in Fig. 6, the folded part 64 extends over and along the outer surface 78 of the first belt member 54. This enables quick adjustment for the user as the user only needs to insert the end portion 68 under the first ring 60 and secure the end portion 68 onto the outer surface 78 of the first belt member 68. The user can adjust the tension of the first belt member 54 by removing the end portion 68 and readjusting on the fly without having to take off the spinal orthosis 50.

According to the depicted example, the outer surface 78 of the first and second belt members 54, 56 includes hook receivable or loop material, and the end portions 68, 70 of the first and second belt members 54, 56 include hook material engageable with the material of the outer surface of the first and second belt members 54, 56. In this variation, the excess lengths of the first and second belt members 54, 56 extending from the rings 60, 62 do not pile up to create pressure points along the circumference of the spinal orthosis 50 directly adjacent the user.

The spinal orthosis 50 is not limited to securing the end portions 68, 70 of the first and second belt members 54, 56, and may be secured along an inner surface of the first and second belt members 54, 56 in a reverse manner to the aforementioned method. The inner surface of the first and second belt members 54, 56 may include hook receivable material, and the end portions 68, 70 are rearranged accordingly with hook material. In this variation, the end portions 68, 70 are located on the inner side of the spinal orthosis 50 and reduce the possibility of leaving excess length of the first and second belt members 54, 56 on the exterior of the spinal orthosis 50.

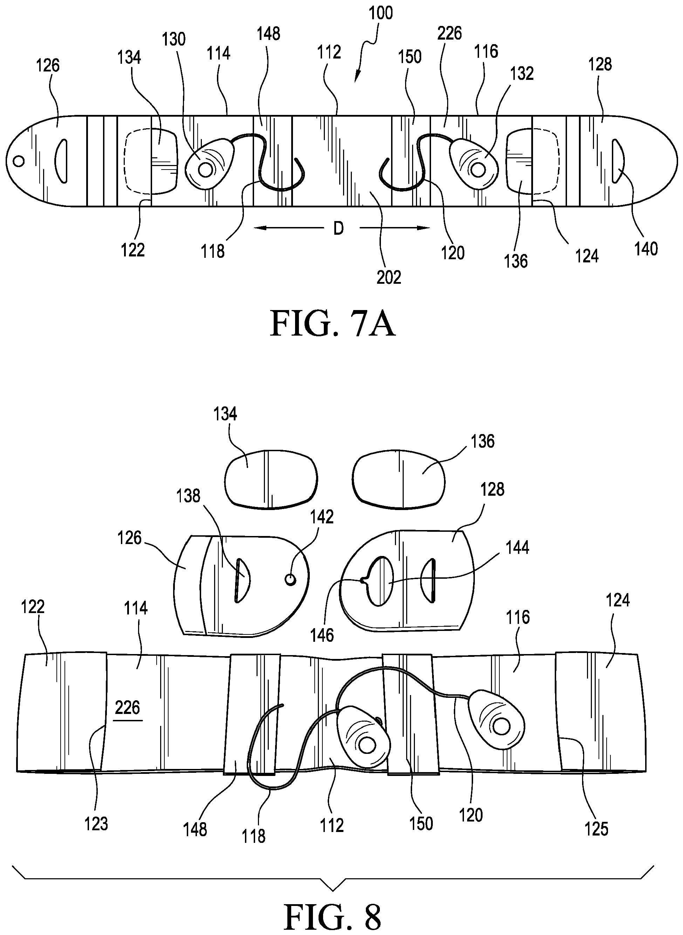

Figs. 7A and 8 show a spinal orthosis 100 including a rear panel 112 having first and second sides. A first belt member 114 has a first end secured to a first side of the rear panel 112 and a second end 123 is configured to have a foldable portion 122 adapted to fold over a surface 226 of the first belt member 114 to reduce a length of the first belt member 114. A second belt member 116 has a first end secured to a second side of the rear panel 112 and a second end 125 configured to have a foldable portion 124 adapted to fold over a surface 226 of the second belt member 116.

While the surface 226 in Figs. 7A and 8 is shown as the outer peripheral surface of the spinal orthosis 100, the spinal orthosis 100 may be configured so the foldable portions 122, 124 go over an inner peripheral surface of the spinal orthosis 100. The outer peripheral surface is the surface defined as being opposite the inner peripheral surface arranged adjacent the anatomy of the user.

The first and second belt members 114, 116 may be removably secured to the rear panel 112 by first and second flaps 148, 150 extending from the first side of rear panel 112, or securable to the first and second belt members 114, 116 and the first and second sides of the rear panel 112. Alternatively, the first ends of the first and second belt members 114, 116 may be permanently secured to the first and second sides of the rear panel 112, such as by stitching or other appropriate means.

First and second patches 134, 136 may secure to the foldable portions 122, 124 of the first and second belt members 114, 116 and a surface 226 of the first and second belt members 114, 116 to maintain an adjusted length of the first and second belt members 114, 116 obtained by selectively arranging the length of the foldable portions 122, 124. Other methods and structures may be used rather than the patches, such as snaps or other locking elements selectively located along at least portions of the length of the first and second belt members 114, 116. The patches 134, 136, however, omit the need for preselecting and limiting the amount of possible lengths as the length of the first and second belt members 114, 116 is made substantially adjustable and is not limited to predetermined size settings.

First and second front closures 126, 128 secure over the foldable portions 122, 124 of the first and second belt members 114, 116. The first and second front closures 126, 128 are arranged to connect to the second ends 123, 125 of the corresponding first and second belt members 114, 116. The first and second front closures 126, 128 form a continuously circumferential loop with the rear panel 112 and the first and second belt members 114, 116 to completely encircle a user's torso and/or other proximate anatomy.

The first front closure 126 includes a locking element 142 defined on a second side and is arranged to engage a corresponding slot 144, 146 defined on a first side by the second front closure 128 for securing the first and second belt members 114, 116 to one another. The slot 144, 146 of the second front closure 128 defines an opening 144 and a keyhole 146 depending at a forward end of the slot 144 proximate the first end of the second front closure 128. When the locking element 142 is inserted into the opening 144, it is directed and slips into the keyhole 146 which is sized and configured closely to the size of the locking element 142 to maintain engagement therewith. The opening 144 is sized greater than the keyhole 146, and on tensioning of the first and second belt members 114, 116 draws the locking element 142 toward the keyhole 146.

The locking element 142 and the slot 144, 146 enables quick and easy locking of the first and second front closures 126, 128, as well as quick and easy removal of the first and second front closures 126, 128 from one another. The locking element 142 and slot 144, 146 likewise require consistent donning and placement of the first and second front closures 126, 128 to one another, and require less strength and force for removal as compared to conventional methods such as using hook and loop systems. While the locking element 142 and slot 144, 146 are preferred, they may be replaced with a hook and loop system with the first front closure 126 including a hook section and the second front closure 128 including a corresponding loop section for engagement with the hook section.

The first and second front closures 126, 128 define first and second pockets 138, 140 along a first side, and are configured and dimensioned for inserting at least a finger thereinto for locating a second end of the first front closure 126 relative to the second end of the second front closure 128.

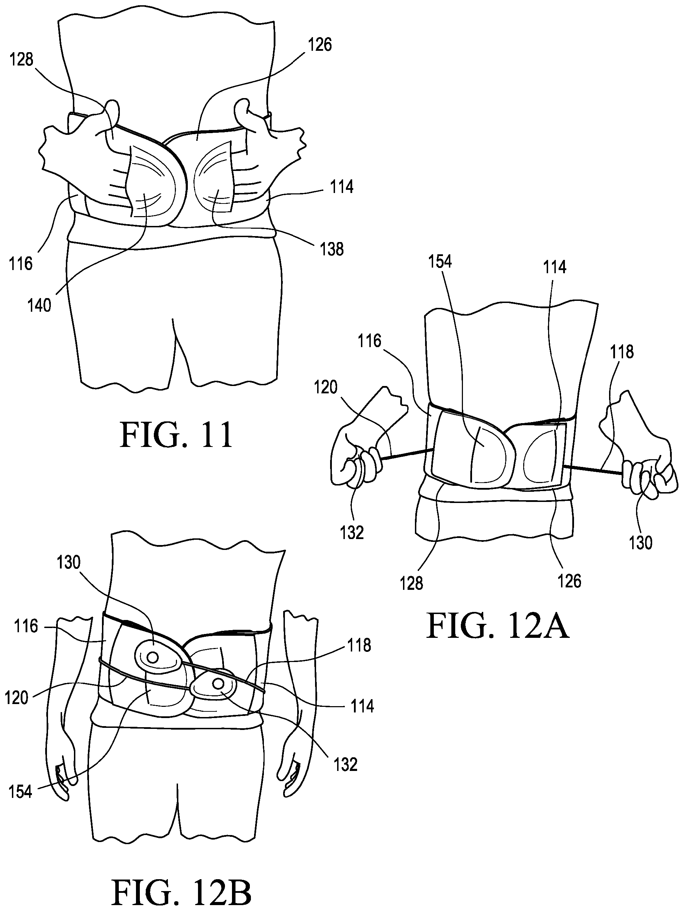

As shown in Fig. 11, the pockets 138, 140 may be arranged to receive a hand of a user so as to remove the necessity of pulling the front closures 126, 128 toward one another, and are beneficial for geriatric users that may have difficulty pulling ends of the first and second front closures 126, 128 with their fingers. The pockets 138, 140 ensure consistent placement of the user's hands for locking the locking element 142 with the slot 144, 146.

The rear panel 112 includes a compression system (not shown) and a stretchable cover 202 extending from the first and second sides. The cover 202 stretches or retracts over a variable distance D according to adjustment of the compression system. The compression system may be arranged in the same manner as discussed in connection with the spinal orthosis example 50. The first and second belt members 114, 116 are formed from a substantially non-stretchable material such that pulling the second ends 123, 125 of the first and second belt members 114, 116 causes the variable distance D to increase, and releasing the second ends 123, 125 causes the cover 202 to retract to a predetermined distance D.

First ends of elongate first and second tensioning elements 118, 120 engage the compression system, and first and second handles 130, 132 secure to second ends of the tensioning elements 118, 120. As shown in Figs. 12A and 12B, the handles 130, 132 may secure to a surface of the first and second belt members 114, 116 and the first and second front closures 126, 128 for selective tensioning of the spinal orthosis by the compression system, as discussed in U.S. patent 8,172,779 or U.S. patent application publication 2014/0207041.

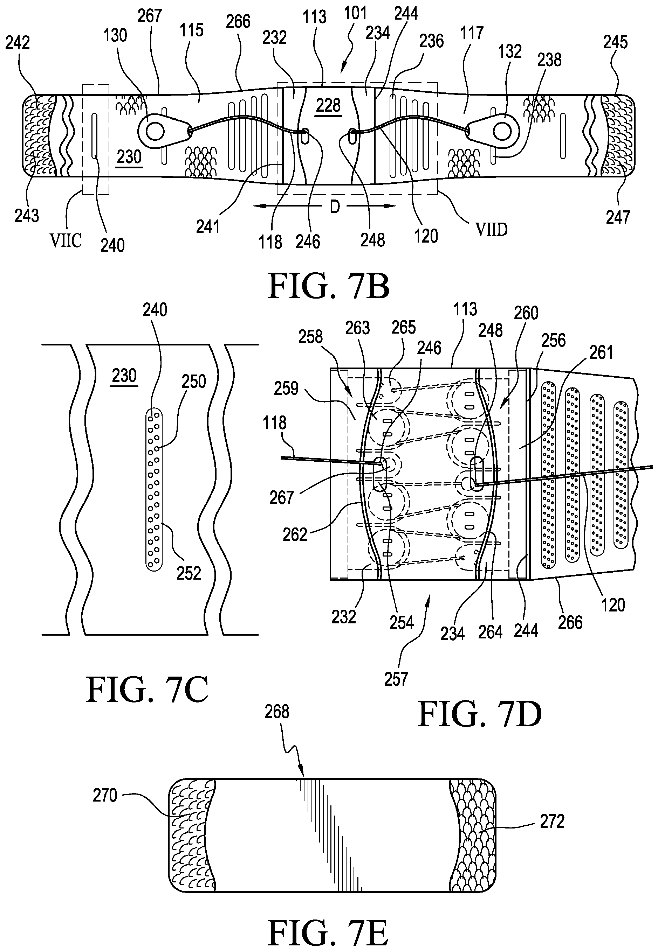

Fig. 7B exemplifies another spinal orthosis example 101 bearing similarity in part to the spinal orthosis 100 of Fig. 7A. In the example of Fig. 7B, the spinal orthosis 101 includes a rear panel 113 having first and second sides. A first belt member 115 has a first end 241 secured to the rear panel 113 and a second end 242 bearing a fastener 243 for securing over a surface 230 of the first belt member. The surface 230 is preferably an outer surface as compared to an inner surface directly facing and adjacent the user. The fastener 243 and the surface 230 preferably form a hook-and-loop fastener attachment system. A second belt member 117 similarly has a first end 244 secured to a second side of the rear panel 113 and a second end 245 with a fastener 247. The second belt member 117 has a same surface 230 as the first belt member 115, and likewise preferably forms a hook-and-loop fastener attachment system with the fastener 247. The spinal orthosis 101 is envisioned as being modified so the fasteners 243, 247 can secure to the inner surface of the first and second belt members 115, 117, with the inner surface having hook receivable material.

As shown in Fig. 7B, both the fasteners 243, 247 of the second ends 242, 245 are located on the same side of the first and second belt members 115, 117. Both the first and second belt members 115, 117 are arranged to be reduced in length and the fasteners 243, 247 secured to the same side of the spinal orthosis on the surface 230. Such an arrangement is different from known spinal orthoses whereby the second side of a first belt member bears hook material, and the first side of a second belt member bears loop material for receiving hook material of the first belt member.

The first and second belt arms 115, 117 preferably have a first contour 266 tapering from first ends 241, 244 from the rear panel 113 and becoming linear in a second contour 267 toward the second ends 242, 245. The first contour 266 enables greater coverage over the posterior body part (i.e., back) of the user which undergoes compression by the spinal orthosis 101. As discussed in connection with Fig. 7D, the spinal orthosis 101 includes a compression system that preferably includes a pulley set. The first contour 266 accommodates the pulley set, whereas the second contour 267 provides a more streamlined and narrow belt configuration where less belt material is required.

Referring to Figs. 7B and 7D, the spinal orthosis 101 includes first and second compression system covers 232, 234 secured to the first ends 241, 244 of the first and second belt members 115, 117, and either extend over the rear panel 113, or connect the first and second belt members 115, 117 thereto. The compression system covers 232, 234 have contours 262, 264 that taper to a center point along the height of the rear panel 113 and flare toward upper and lower peripheral edges of the rear panel 113. The arrangement of the compression system covers 232, 234 is provided in part to maximize stretching of the rear panel 113 at the center point.

The rear panel 113 variably extends the distance between the compression system covers 232, 234, as noted above in connection with the example of Fig. 7A. The rear panel 113 can be made from an elastic material, such as Spandex. The rear panel 113 defines openings 246, 248 through which the tensioning members 118, 120 extend from the compression system for selective securement onto the surface 230 of the belt members 115, 117, by the first and second handles 130, 132. The openings 246, 248, are preferably reinforced by a tape 254 laminated or welded to the material forming the rear panel 113.

The first and second belt members 115, 117 and the compression system covers 232, 234 are preferably constructed from a sheet or laminated sheets of loop material. Unlike in many prior art spinal orthoses, the first and second belt members 115, 117 of the spinal orthosis in Fig. 7B are substantially supple to enable folding of the first and second belt members 115, 117 for resizing thereof. For example, the construction of the first and second belt members 115, 117 may comprise at least two sheets of loop material formed by knitted fabric laminated to one another without other types of intermediate materials, such as spacer fabric in the prior art. This construction results in a substantially thin belt member while having some compressibility due in part to the loop material, and with both inner and outer surfaces of the first and second belt members 115, 117 as having hook-receivable material by the loop material structure. Because both the inner and outer surfaces of the first and second belt members 115, 117 is preferably constructed from hook-receivable material, there is a significant amount of space, such as along the foldable portions 122, 124 and outside the foldable portions 122, 124, for securing the front closures 126, 128 to the first and second belt members 115, 117. Unlike the rear panel 113, the first and second belt members 115, 117 are preferably not elastic or stretchable.

Referring to Figs. 7B and 7C, the first and second belt members 115, 117 preferably include perforated areas. For example, a first set of perforations 236 are defined proximate to the first ends 241, 244 of the first and second belt members 115, 117. The first set of perforations 236 cascade from the first ends 241, 244 and diminish in height substantially according to the taper of the first contour 266 of the first and second belt members 115, 117. Additional sets or individual perforations may be formed along the length of the first and second belt members 115, 117, such as a second set of perforations 238 generally located at a mid-length of the first and second belt members 115, 117, and a third set of perforations 240 generally located near the second ends 242, 245 of the first and second belt members 115, 117.

Fig. 7C shows section VIIC of Fig. 7B, which shows particularly that each of the sets of perforations may define a plurality of perforations 250 preferably formed through the thickness of the first and second belt members 115, 117, and are distinguishable from porosity, weave, and structure of the first and second belt members 115, 117 in that the perforations 250 have a predetermined shape. The first and second belt members 115, 117 may be embossed or have a reduced thickness area 252 about the sets of perforations. The reduced thickness area 252 is preferably oriented parallel to a height of the first and second belt members 115, 117, and may extend from one side or both sides relative to the thickness of the first and second belt members 115, 117. The reduced thickness area 252 is not limited to being arranged parallel to the height of the spinal orthosis 101, but may be arranged in other desirable directions inclusive of being parallel to the length of the belt members. As depicted, the reduced thickness areas 252 preferably have a length shorter than the respective height of the first and second belt members 115, 117 whereat the reduced thickness areas 252 are located in order to maintain structural integrity of the first and second belt members 115, 117, although the reduced thickness areas 252 may extend the entirety of the height of the first and second belt members 115, 117.

The sets of perforations 250 and the reduced thickness areas 252, either alone or in combination, may facilitate bending of the first and second belt members 115, 117 and offer areas of enhanced breathability without substantially hindering the structural integrity of the first and second belt members 115, 117. As the first and second belt members 115, 117 are substantially thin, they may have a thickness of 1 to 5 mm, and more preferably a thickness of 1.5 to 3.0 mm. The thickness at the reduced thickness areas 252 may have a thickness of 0.5 to 1.5 mm.

Fig. 7D exemplifies the compression system 257 (shown in section VIID of Fig. 7B) as having first and second pulley sets 258, 260. Each of the pulley sets 258, 260 define first and second panels 259, 261 which include an array of pulleys 263 about which both the first and second tensioning members 118, 120 rotate about. A first end of the tensioning members 118, 120 is anchored at an anchor 265 to one of the first and second panels 259, 261, and extend through a respective guide 267 likewise formed on the first and second panels 259, 261.

The first and second panels 259, 261 are considered "single" in that they include pulleys 263 for both the first and second tensioning members 118, 120, whereby some pulleys 263 are individually dedicated for the first and second tensioning members 118, 120. For example, the first tensioning member 118 is anchored to anchor 267 on the first panel 259, and extends through a first pulley on the second panel 261, is routed to a first pulley on the first panel 259, and back to a second pulley on the second panel 261, and finally then routed to the guide 267 on the first panel 259 before extending from the exit hole 246 defined by the rear panel 113. The second tensioning member 120 is similarly routed between the first and second pulley sets 258, 260 as the first tensioning member 118, but is arranged about different pulleys located below the entirety of the pulleys 263 about which the first tensioning member 118 extends.

From the foregoing, it follows that adjustment of either of the first and second tensioning members 118, 120 will adjust the first and second panels 259, 261 to some degree since all of the pulleys 263 are carried by the first and second panels 259, 261. The length of the rear panel 113 and therefore the distance between the first and second panels 259, 261 will adjust according to adjustment of at least one of the first and second tensioning members 118, 120.

The first and second panels 259, 261 are respectively secured to the first ends 241, 244 of the first and second belt members 115, 117 at an edge reinforcement or interface 256, along with ends of the rear panel 113 and the compression system covers 232, 234, which serve in part to reinforce the substantially thin and elastic material of the rear panel 113. The edge interface 256 may comprise stitching of the aforementioned components together as a unitary interface, or some components may be laminated or welded together or with others stitched to one another.

Fig. 7E depicts a belt extender 268 that is arranged for extending a length of one of the first and second belt members 115, 117. The belt extender 268 includes a first end having a fastener 270, such as hook material, and a second end having a fastener 272, such as loop material. The fastener 272 at the second end is shaped to correspond to the second ends 242, 245 of the first and second belt members 115, 117, and the fasteners 243, 247 disposed thereon for engagement therewith. The fastener 270 at the first end takes the place of the fasteners 243, 247 at the first ends of the first and second belt members 115, 117 when the second fastener 272 engages the first and second belt members. The belt member between the first and second fasteners 270, 272 may be formed similarly as the same construction as the first and second belt members 115, 117. The first fastener 270 is preferably located on a different side of the belt extender 268 from the second fastener 272, although the belt extender 268 may be adapted so the first and second fasteners 270, 272 are on the same side.

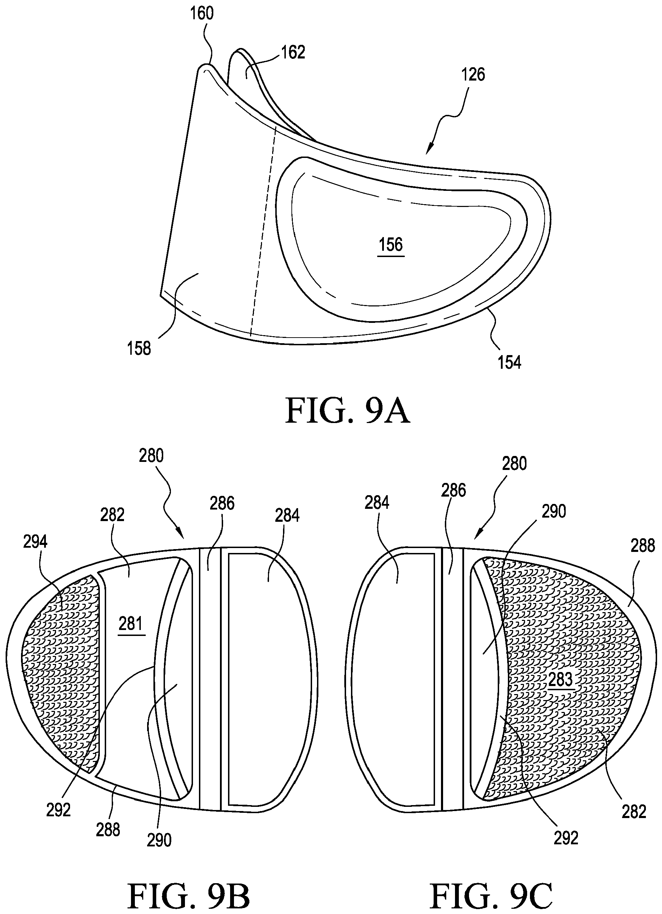

Referring to Fig. 9A, an example of the first front closure 126 defines first and second clamping sections 160, 162 at a rear section 158 arranged to removably secure to opposed sides of the foldable portion 122 of the first belt member 114 in Fig. 8. The first front closure 126 includes material 156 at least at the forward end 154 for receiving the handles in Fig. 8. The material 156 may spread across both the forward and rear sections 154, 158. The clamping sections 160, 162 may be modified over the description in U.S. patent application publication 2014/0081189, published March 20, 2014, to accommodate foldable portions of a belt member rather than severed ends of a belt member.

Figs. 9B and 9C depict another example of a front closure 280 defining a first or leading section 282 for securing to a corresponding front closure on another side of the belt members or a belt member, a second or trailing section 284 for securing to the belt members, and a third section or divider 286 located between the first and second sections 282, 284. A peripheral portion 288 preferably extends about the first and second sections 282, 284, and is divided by the third section 286. The peripheral binding 288 may define a tapered and/or soft edge.

Both first and second sides 281, 283 of the front closure 280 define pockets 290 delimited by a pocket periphery 292. The first side 281 includes a fastener 294, preferably in the form of hook material but may include other known types of fasteners. The second side 283 likewise includes a fastener, preferably in the form of hook material formed by a surface of the front closure 280. The fastener 294 may engage the surface of the second side 283 or the material surface of the belt members.

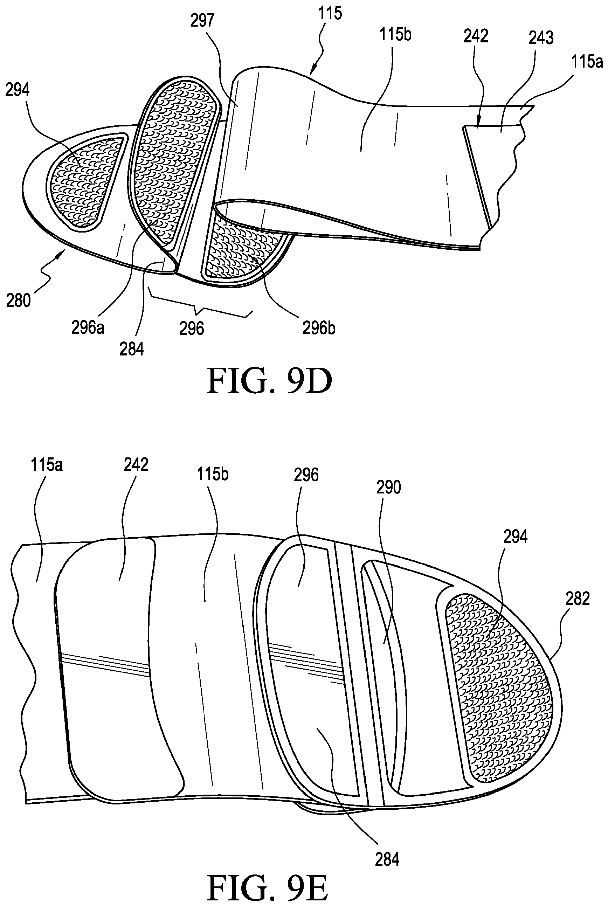

Figs. 9D and 9E exemplify how the front closure 280 secures to the belt members. The front closure 280 defines a clamp 296 at the second section 284, and is preferably defined by first and second flaps 296a, 296b bearing hook material that secures onto opposed sides of the first belt member 115. The flaps 296a, 296b preferably extend and open to the third section 286 from which the flaps 296a, 296b hinge.

The second end 242 of the first belt member 115 folds over itself at a selected length at a crease 297 with a first belt member segment 115a extending between the rear panel (not shown) to the crease 297 and a second belt member segment 115b extending from the crease 297 to the second end 242. The fastener 243 at the second end 242 secures to the first belt member segment 115a. The flaps 296a, 296b secure to the first belt member 115 at and over the crease 297 by engaging a hook surface of the first belt member 115.

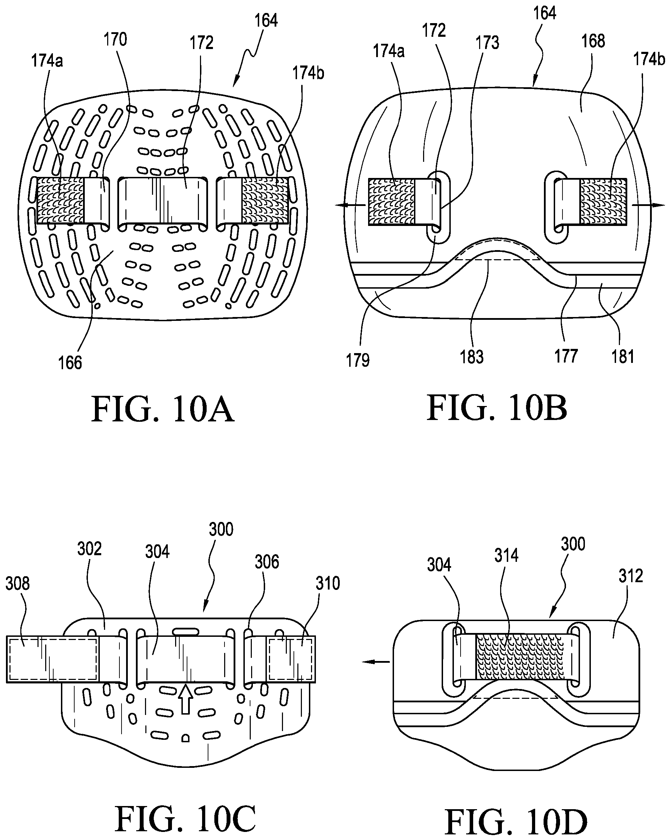

Figs. 10A and 10B depict a supplementary panel 164 having a connector 172, such as a strap, arranged for removably securing to the compression system covers in the spinal orthosis of Fig. 7B or the belt members 115, 117 by fasteners 174a, 174b. The connector 172 is preferably elastic so that it can stretch or contract according to displacement of the variable clearance between the pulley sets 258, 260 and rear panel 113. The connector 172 is slidably retained to the supplementary panel 164 by at least one slot 170 through which the connector 172 extends. The at least one slot 170 enables the connector 172 to stretch outwardly or away from the supplementary panel 164 (as shown with the arrows) and contract without detriment to the supplementary panel 164.

The supplementary panel 164 preferably includes a plate 166 that may be apertured and is arranged to generally conform in geometry to a lower back or lumbar region for a user. The plate 164 preferably defines the at least one slot 170. The plate 166 may be substantially rigid or semi-rigid, or alternatively flexible but upon compression against a body part of a user is rendered rigid.

The supplementary panel 164 includes a cover 168 which substantially or fully encases the plate 166. In the example of Fig. 10B, the cover 168 includes at least one slot 173 generally corresponding in location to the at least one slot 170 defined by the plate 166. The cover 168 defines a slit 177 enabling insertion and extraction of the plate 166 into and from the cover 168. The slit 177 preferably is releasably closeable with a fastener 183 keeping opposed sides of the cover 168 at the slit 177 secured to one another. Edge reinforcement 179 is provided about the at least one slot 173 and additional edge reinforcement 181 may be provided about the slit 177. The edge reinforcement 179, 181 may be a reinforcing film laminated or welded to the material forming the cover 168.

Figs. 10C and 10D depict an anterior panel 300 arranged for securing to belt members, particularly near or at the second ends thereof. The anterior panel 300 includes a substantially rigid shell 302 and a connector 304 extending through at least one slot 306 formed by the substantially rigid shell 302. The connector 304 is preferably inelastic and has opposed ends 308, 310 which secure to one another to form a loop. When secured to one another in a loop, the opposed ends 308, 310 bear on an outer surface thereof a hook material 314 for securing to the first and second belt members 115, 117. The anterior panel 300 may include a cover 312 arranged similarly to the cover for the supplementary panel 164.

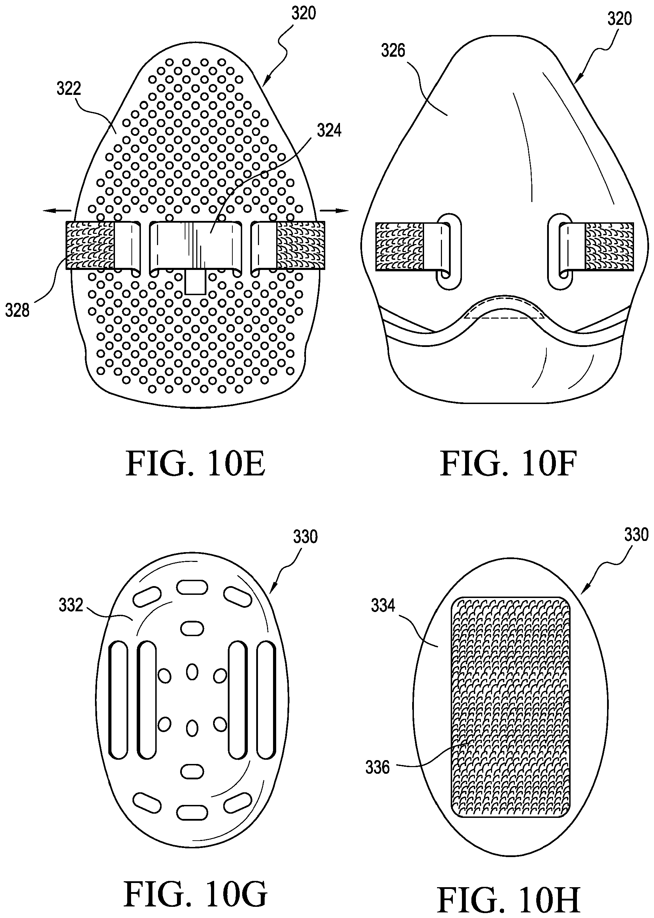

Figs. 10E and 10F illustrate a posterior panel 320 having a rigid shell 322 and a connector 324 bearing fasteners 328 similar to the supplementary panel 164 of Figs. 10A and 10B for securing to the compression system covers 232, 234. The posterior panel 320 likewise includes a cover 326 arranged similarly to the cover in the supplementary panel 164, including a slit for insertion and removal of rigid shell 322

Figs. 10G and 10H depict a lateral panel 330 having a substantially rigid shell 332, and a removable cover 334 bearing a fastener 336. The lateral panel 330 is arranged for securing to the belt members between their first and second ends for providing lateral side support for the spinal orthosis.

The panels 164, 300, 320, 330 may be used as needed to achieve correct fit and positioning. The rigid posterior panel 320 should be centered on the spine with the bottom of the rigid posterior panel 320 at approximately the sacroiliac joint. The rigid lateral panels 330 should be placed on landing zones on the belt arms, which may be formed by the embossed or reduced thickness regions. The anterior panel 300 should be centered on the abdomen with the bottom edge just above the symphysis pubis while still allowing the patient to sit comfortably.

The panels 164, 300, 320, 330 can be modified as necessary to optimize patient fit and comfort by removing them from their respective sleeves and adjusting the panels 164, 300, 320, 330 with a heat gun and/or a trimming device. The spinal orthosis is a modular system and can be customized to the needs of the user. The panels 164, 300, 320, 330 can be added or removed depending on motion restriction and compression desired throughout a rehabilitation period. Any of the panels 164, 300, 320, 330 described herein may include any of the features of the plates or panels described in U.S. patent application publication 2014/0081189.

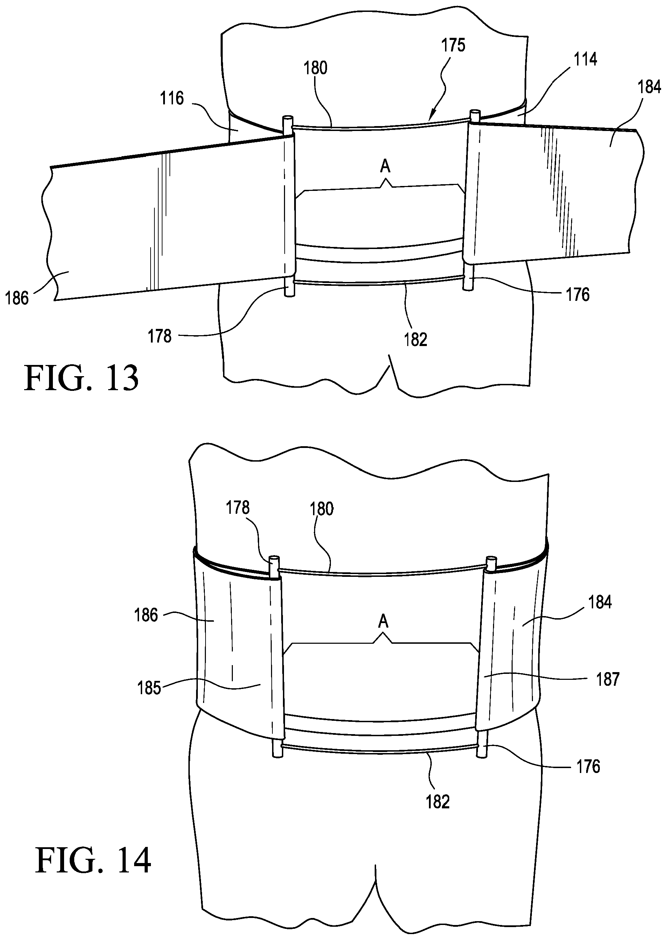

Figs. 13 and 14 depict a kit and method for sizing a lumbar belt having features of the spinal orthosis 100 of Fig. 7A by adjusting relative lengths of the first and second belt members 114, 116. A sizing device 175 has first and second positioning elements 176, 178 for establishing a clearance defined by a predetermined distance (A) between the second ends of the first and second belt members 114, 116. The first and second positioning elements are first and second rods 176, 178 spaced apart by first and second cords 180, 182 each having a length (A) extending the predetermined distance between the first and second rods 176, 178.

As shown, the second ends of the first and second belt members 114, 116 are inserted and pulled over the first and second positioning elements 176, 178 to determine a resized length of the first and second belt members 114, 116 between the rear panel 112 and the first and second positioning elements 176, 178. The patches are used to secure the first and second foldable portions 184, 186, and pulled and tensioned about the first and second positioning elements 176, 178. The first and second belt members 114, 116 are resized to have a new length between the rear panel 112 and an end of the foldable portions 184, 186.

Of note, the first and second foldable portions 184, 186 bear fastener material, as in the fasteners 243, 247 in the example of Fig. 7B, so folded portions 185, 187 can extend outwardly (or inwardly) and be tensioned about the positioning elements 176, 178 in the same manner, such as extending from inside and over the positioning elements 176, 178, as shown in Fig. 13. By having both of the foldable portions 184, 186 extending in the same orientation, the user can generally uniformly tension and size the length of the foldable portions 184, 186, as the first and second belt members 114, 116 will likewise have the same length after sizing of the foldable portions 184, 186.

The first and second front closures 126, 128 are secured on the resized first and second belt members 114, 116, and the distance needed to accommodate the first and second front closures 126, 128 is assured by the length of the cords 180, 182. An advantage to this kit and method is that the first and second belt members 114, 116 are sized without a need for trimming. The first and second belt members 114, 116 can be resized according to different needs of a user, and any changes to the first and second belt members 114, 116 can be redone, reversed and resized without the use of complicated buckles or fasteners.

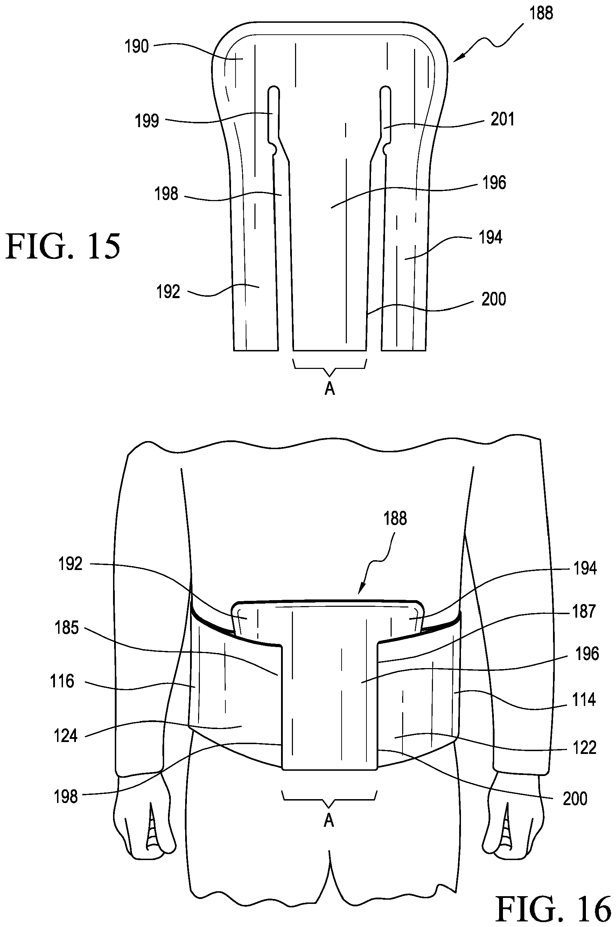

Turning to Figs. 15 and 16, another kit and method is provided for resizing the relative belt lengths of the first and second belt members 114, 116 for a user. In this kit, the sizing device 188 is a rigid board having a main portion 190 and the first and second positioning elements 192, 194 extend from the main portion 190. The first and second positioning elements 192, 194 are spaced apart by a center section 196 with a width or predetermined distance (A) forming the predetermined distance discussed above in connection with the sizing device 175. First and second grooves 198, 200 are formed by the board 188, and separate the first and second positioning elements 192, 194, respectively, from the center section 196. Each of the first and second grooves 198, 200 include upper grooves 199, 201 arranged to pinch the first and second belt members 114, 116 to better retain them relative to the sizing device 188.

In the method shown in Fig. 16, sizing the lumbar belt using the sizing device 188 includes placing the first and second belt members 114, 116 through the first and second grooves 198, 200, and drawing the foldable portions 122, 124 back toward the rear panel 112 so they are tensioned against the first and second positioning elements 192, 194 and secured using various means described above. The folded portions 185, 187 are positioned apart from one another by the width (A) for placement of the first and second front closures 126, 128.

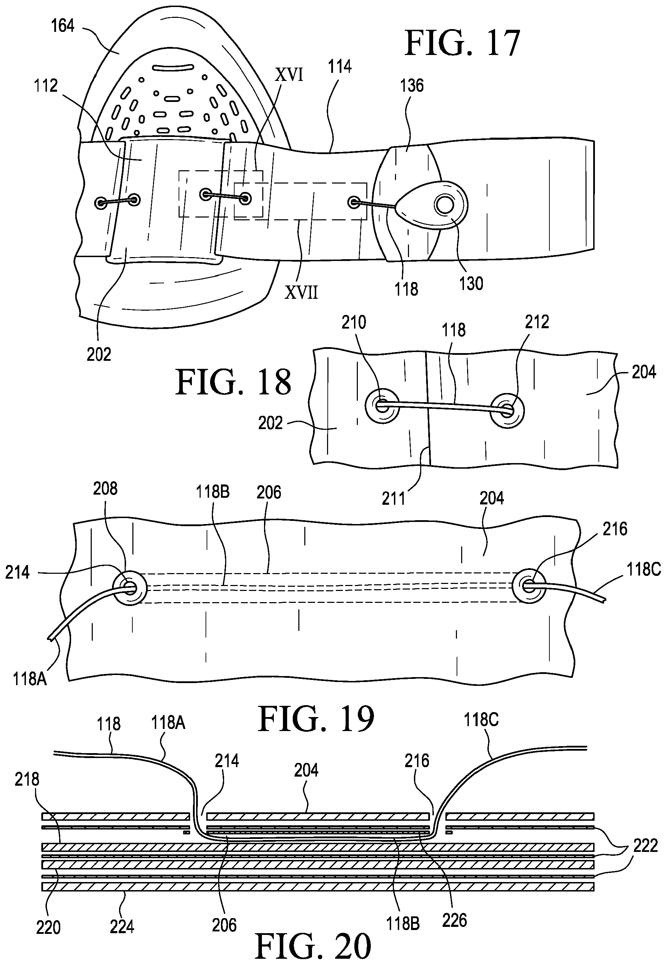

Figs. 17-20 illustrate how the belt members 114, 116 may have means for concealing various length segments of the tensioning element 118. For example, the first belt member 114 may form a channel 206 including first and second openings 214, 216 for concealing the tensioning element 118, in the form of a cable. The channel 206 is formed between first and second peripheral surfaces 204, 224 of the belt member 114.

Fig. 18 depicts a section XVI in Fig. 17 of the rear panel 112 having a panel material 202 surrounding the compression system, as discussed above. The panel material 202 may be preferably stretchable to accommodate activation of the closure system. The cable 118 extends from interior of the panel material 202 and exits from aperture 210. The cable 118 spans where the first belt member 114 and panel material 202 join at joint 211, and enters the first belt member 114 at an aperture 212. In this example, the panel material 202 and the belt are constructed differently, although the rear panel 112 and first belt member 114 at the joint between the rear panel 112 and first belt member 114 may be constructed to permit the cable 118 to extend across the joint in a concealed manner.

Referring to Fig. 19, a section XVII in Fig. 17 is shown. The cable 118 has a first portion 118A outside the channel 206 and proximate the first opening 214. A second portion 118B extends through the first opening 214 and within the channel 206. A third portion 118C extends from the second opening 216 and outside the channel 206. For any of the openings 214, 216, an eyelet 208 may be provided to reinforce the material about the apertures.

Referring to Fig. 20, the belt member 114 may include a core including first and second core layers 218, 220 or a single substrate, and first and second layers 204, 224 forming the first and second peripheral surfaces such as outer and inner surfaces of the first belt member 114, respectively, and on opposed sides of the first and second core layers 218, 220. A third layer 226 is secured to the first layer 204 whereby the channel 206 is located between the third layer 226 and the first and second core layers 218, 220. The third layer 226 generally only extends between the first and second openings 214, 216 to reinforce the first layer and facilitate sliding of the cable 118.

The first and second core layers 218, 220, and the first, second and third layers 204, 224, 226 are laminated to one another with the exception of the third layer 226 which is not laminated to the first and second core layers 218, 220 to permit opening of the channel 206. The first layer 204 is laminated to the first and second core layers 218, 220 outside of the third layer 226. Adhesive layers 222 may be used to secure the various layers to one another through lamination.

The channel 206 is advantageous in that it serves as a retainer of the cable 118, and reduces the amount of cable 118 exposed to be tangled or snagged on external elements. The channel 206 moves the exit point of the cable 118 forward on the first and second belt members 114, 116 which makes it easier for the user to find and see the cable 118. Various channels may be provided along the length of the first and second belt members 114, 116 or a single, long channel may be formed.

The solution provided above eliminates a need for stitching, and instead relies on lamination of the layers. The channel 206 is not limited to the solution provided above, and may indeed include channels formed by stitching of the layers of the belt members.

Figs. 21A-22B exemplify a closure embodiment 400 for securing to the aforementioned belt members. Unlike the embodiment of the front closure in Figs. 9B and 9C, the closure 400 is adapted to wrap about the belt member in manner that significantly resists shearing from the belt member. The closure 400 defines an inner contour 426 having a generally arcuate shape for better conforming to a curvature of a user's waist, and upper and lower notches 416 for better securing to the belt member. The closure 400 defines an outer contour with a tapering profile 414 to facilitate stripping the closure 400 from the belt member or an opposed closure.

The closure 400 includes on an outer surface O a fastener tab 402 separated from a central portion 406 by a central folding portion 410. The outside surface also includes upper and lower portions 418, 422 spaced apart by the central portion 406 and folding portion 412 located between the upper and lower portions 418, 422 and the central portion 406. The upper and lower portions 418, 422, and the central portion 406 may be formed from a fabric, such as a brushed loop material.

Inside surface I has upper and lower fastener portions 420, 424, a central fastener portion 408, and a fastener tab 404, which are likewise spaced apart by the folding portion 412 and the central folding portion 410. The upper and lower fastener portions 420, 424 and the central fastener portion 408 correspond to the upper and lower portions 418, 422 and the central portion 406 of the outside surface O. The upper and lower fastener portions 420, 424 are adapted to wrap over an edge of the belt member by the folding portion 412 apart from the central portion 408 which secures to an opposite side of the belt member from the upper and lower fastener portions 420, 424. The fastener tab 404 is arranged to secure to a securing portion of one of the closures and corresponding on an opposed side to the fastener tab 402 on an opposite belt member or over the surface of the belt member.

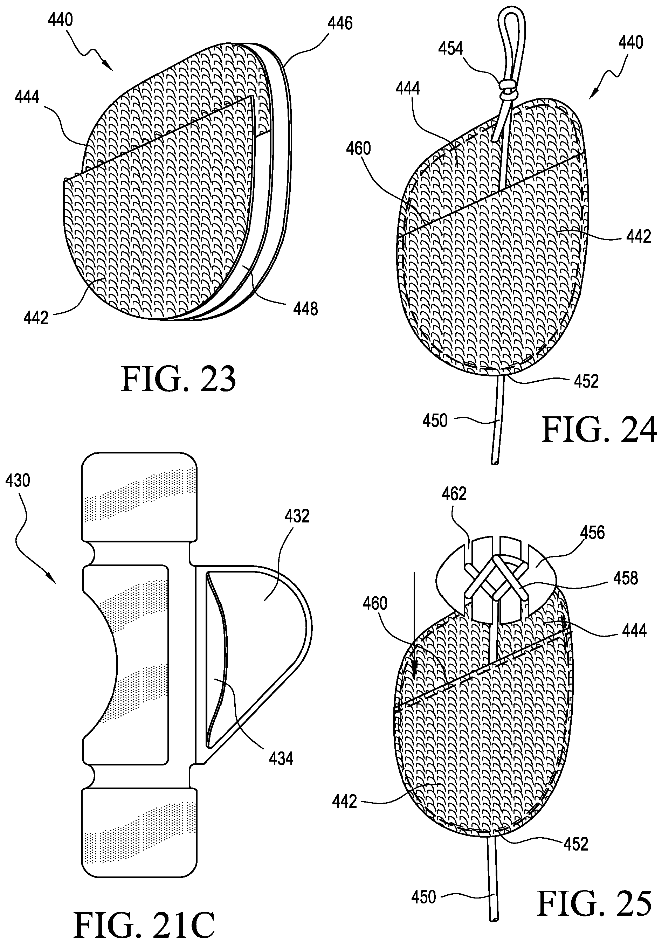

Fig. 21C shows a closure 430 with a pocket 432 formed or secured thereon. The pocket 432 may include an opening 434 for insertion of fingers for assisting in closing the belt members and securing the closure 430 thereon or on another closure.

Figs. 22A and 22B exemplify how the closure is adapted to fold over the belt member.

Figs. 23 and 24 exemplify a pull tab embodiment 440 having a first layer 442 defining an outer side with hook material and an inner side with non-hook material, such as broken loop material. A second layer 444 includes a hook material along a least a portion of the outer side, and a region 448 without such hook material on the outer side. The first layer 442 is arranged shorter than the second layer 444, and defines a pouch 460 therewith such that the region 448 without hook material of the second layer 444 is located within the pouch 460. A third layer 446 is secured to the second layer 444 and may be formed by a rubber material or other suitable material for enabling easy gripping of the pull tab 440.

Fig. 24 shows how an opening 452 is formed between the first and second layers 442, 444 at an end opposite the pouch 460 for insertion of a tensioning element or cable 450 therethrough. The cable 450 is pulled through the opening 452 and out from the pouch 460. The cable 450 may be provided with a knot 454 or otherwise configured to prevent slippage of the cable 450 through the opening 452 when the cable 450 is tensioned, and arranged to enable resizing of the cable 450 according to varied size changes of the belt members.

Fig. 25 shows an alternative for resizing the cable 450 in that a cable retainer 456 defines a plurality of slots 462 for wrapping a segment 458 of the cable 450 thereabout. The cable retainer 456 can be retained within the pouch 460 and pulled out depending on the desirability to resize the length of the cable 450.

While the foregoing embodiments have been described and shown, alternatives and modifications of these embodiments, such as those suggested by others may be made to fall within the scope of the disclosure as set out in the claims. While the embodiments herein have been described in combination with a spinal orthosis, it will be understood that the principles described may be extended to other types of orthopedic and prosthetic devices.

## Claims

1. A spinal orthosis (100), comprising:
- a rear panel (112) having first and second sides;
- a first belt member (114) having a first end secured to the first side of the rear panel (112) and a second end at an opposite side of the first end;
- a second belt member (116) having a first end secured to the second side of the rear panel (112) and a second end at an opposite side of the first end;
- first and second front closures (400) which are removably securable to the second ends of the first and second belt members (114, 116), respectively, and arranged for removably securing to one another to form a continuously circumferential loop with the rear panel (112) and the first and second belt members (114, 116);
**characterized in that** the first front closure (400) has:
- upper and lower clamping portions (420, 424) separated from one another and located on a same side of the first front closure (400), the upper and lower clamping portions (420, 424) arranged to secure to a same surface of the first belt member (114);
- and a central clamping portion (408) spaced apart by folding portions (412) from the upper and lower clamping portions (420, 424) such that the upper and lower clamping portions (420, 424) are arranged to fold about the folding portions (412) and to wrap over an edge of the belt member (114) to face the central clamping portion (408)

2. The spinal orthosis (100) of claim 1, wherein the upper and lower clamping portions (420, 424) and the central clamping portion (408) define hook material engageable with a surface of the first belt member (114).

3. The spinal orthosis (100) of claim 1, wherein the first front closure (126) defines a pocket (290) along a first side, the pocket (290) configured and dimensioned for inserting at least a finger thereinto for locating a second end of the first front closure (126) relative to the second end of the second front closure (128).

4. The spinal orthosis (100) of claim 1, further comprising a closure system and at least one tensioning element (450) with a handle (440), the at least one tensioning element (450) extending from the closure system and the at least one tensioning element (450) is adjustable in length from the closure system relative to the handle (440) and securable thereto.

5. The spinal orthosis (100) of claim 4, wherein the handle (440) defines a pouch (460) into which a first end portion of the tensioning element (450) is retained.

6. The spinal orthosis (100) of claim 5, wherein the tensioning element (450) includes a cable retainer (456) at the first end portion receivable into the pouch (460).

7. The spinal orthosis (100) of claim 6, wherein the handle (440) defines an opening (452) through which the tensioning element (450) slides, the retainer (456) preventing sliding of the tensioning element (450) in a first direction.

8. The spinal orthosis (100) of claim 4, wherein the handle (440) defines a first surface (408) having a hook material arranged to engage a surface of the first belt member (114) and a second surface (406) forming a traction feature.

9. The spinal orthosis (100) of any one of claims 1 to 8, wherein the closure (400) defines an inner contour (426) having a generally arcuate shape.

10. The spinal orthosis (100) of any one of claims 1 to 9, wherein the closure (400) defines upper and lower notches (416).

## Patentansprüche

1. Wirbelsäulenorthese (100), umfassend:
- eine rückseitige Platte (112) mit einer ersten und einer zweiten Seite;
- ein erstes Gurtteil (114) mit einem an der ersten Seite der rückseitigen Platte (112) gesicherten ersten Ende und einem zweiten Ende an einer gegenüberliegenden Seite des ersten Endes;
- ein zweites Gurtteil (116) mit einem an der zweiten Seite der rückseitigen Platte (112) gesicherten ersten Ende und einem zweiten Ende an einer gegenüberliegenden Seite des ersten Endes;
- einen ersten und zweiten vorderseitigen Verschluss (400), die jeweils lösbar an den zweiten Enden des ersten und zweiten Gurtteils (114, 116) gesichert werden können und zum lösbaren Sichern aneinander, um eine kontinuierlich umlaufende Schlaufe mit der rückseitigen Platte (112) und dem ersten und zweiten Gurtteil (114, 116) zu bilden, angeordnet sind;
**dadurch gekennzeichnet, dass** der erste vorderseitige Verschluss (400) aufweist:
- einen oberen und unteren Klemmabschnitt (420, 424), voneinander getrennt und auf einer gleichen Seite des ersten vorderseitigen Verschlusses (400) befindlich, wobei der obere und untere Klemmabschnitt (420, 424) angeordnet sind, um eine gleiche Oberfläche des ersten Gurtteils (114) zu sichern;
- und einen mittleren Klemmabschnitt (408), von dem oberen und unteren Klemmabschnitt (420, 424) durch Faltabschnitte (412) beabstandet, sodass der obere und untere Klemmabschnitt (420, 424) angeordnet sind, um über die Faltabschnitte (412) zu klappen und sich über einen Rand des Gurtteils (114) zu wickeln, um dem mittleren Klemmabschnitt (408) zugewandt zu sein

2. Wirbelsäulenorthese (100) nach Anspruch 1, wobei der obere und untere Klemmabschnitt (420, 424) und der mittlere Klemmabschnitt (408) mit einer Oberfläche des ersten Gurtteils (114) in Eingriff bringbares Hakenmaterial definieren.

3. Wirbelsäulenorthese (100) nach Anspruch 1, wobei der erste vorderseitige Verschluss (126) entlang einer ersten Seite eine Tasche (290) definiert, wobei die Tasche (290) zum Einführen mindestens eines Fingers dorthinein zum Lokalisieren eines zweiten Endes des ersten vorderseitigen Verschlusses (126) relativ zu dem zweiten Ende des zweiten vorderseitigen Verschlusses (128) konfiguriert und bemessen ist.

4. Wirbelsäulenorthese (100) nach Anspruch 1, weiter umfassend ein Verschlusssystem und mindestens ein Spannelement (450) mit einem Griff (440), wobei das mindestens eine Spannelement (450) sich von dem Verschlusssystem erstreckt und das mindestens eine Spannelement (450) in der Länge von dem Verschlusssystem relativ zu dem Griff (440) einstellbar ist und daran gesichert werden kann.

5. Wirbelsäulenorthese (100) nach Anspruch 4, wobei der Griff (440) einen Beutel (460) definiert, in dem ein erster Endabschnitt des Spannelements (450) zurückgehalten wird.

6. Wirbelsäulenorthese (100) nach Anspruch 5, wobei das Spannelement (450) eine Kabelhalterung (456) an dem ersten Endabschnitt beinhaltet, die in den Beutel (460) aufgenommen werden kann.

7. Wirbelsäulenorthese (100) nach Anspruch 6, wobei der Griff (440) eine Öffnung (452) definiert, durch die das Spannelement (450) gleitet, wobei die Halterung (456) das Gleiten des Spannelements (450) in eine erste Richtung verhindert.

8. Wirbelsäulenorthese (100) nach Anspruch 4, wobei der Griff (440) eine erste Oberfläche (408) mit einem Hakenmaterial definiert, das angeordnet ist, um in eine Oberfläche des ersten Gurtteils (114) und eine zweite Oberfläche (406) einzugreifen, die ein Traktionsmerkmal bildet.

9. Wirbelsäulenorthese (100) nach einem der Ansprüche 1 bis 8, wobei der Verschluss (400) eine innere Kontur (426) mit einer allgemein bogenförmigen Form definiert.

10. Wirbelsäulenorthese (100) nach einem der Ansprüche 1 bis 9, wobei der Verschluss (400) obere und untere Aussparungen (416) definiert.

## Revendications

1. Orthèse vertébrale (100), comprenant :
- un panneau arrière (112) ayant des premier et deuxième côtés ;
- un premier élément de sangle (114) ayant une première extrémité fixée au premier côté du panneau arrière (112) et une deuxième extrémité au niveau d'un côté opposé de la première extrémité ;
- un deuxième élément de sangle (116) ayant une première extrémité fixée au deuxième côté du panneau arrière (112) et une deuxième extrémité au niveau d'un côté opposé de la première extrémité ;
- des première et deuxième fermetures avant (400) qui peuvent être fixées de manière amovible aux deuxièmes extrémités des premier et deuxième éléments de sangle (114, 116), respectivement, et agencées pour se fixer de manière amovible l'une à l'autre pour former une boucle circonférentielle continue avec le panneau arrière (112) et les premier et deuxième éléments de sangle (114, 116) ;
**caractérisée en ce que** la première fermeture avant (400) a :
- des parties de serrage supérieure et inférieure (420, 424) séparées l'une de l'autre et situées sur un même côté de la première fermeture avant (400), les parties de serrage supérieure et inférieure (420, 424) étant agencées pour se fixer à une même surface du premier élément de sangle (114) ;
- et une partie de serrage centrale (408) espacée par des parties de pliage (412) des parties de serrage supérieure et inférieure (420, 424) de sorte que les parties de serrage supérieure et inférieure (420, 424) soient agencées pour se plier autour des parties de pliage (412) et pour s'enrouler sur un bord de l'élément de sangle (114) pour faire face à la partie de serrage centrale (408).

2. Orthèse vertébrale (100) de la revendication 1, dans laquelle les parties de serrage supérieure et inférieure (420, 424) et la partie de serrage centrale (408) définissent un matériau de crochet pouvant s'engager avec une surface du premier élément de sangle (114).

3. Orthèse vertébrale (100) de la revendication 1, dans laquelle la première fermeture avant (126) définit une poche (290) le long d'un premier côté, la poche (290) étant configurée et dimensionnée pour y insérer au moins un doigt pour localiser une deuxième extrémité de la première fermeture avant (126) par rapport à la deuxième extrémité de la deuxième fermeture avant (128).

4. Orthèse vertébrale (100) de la revendication 1, comprenant en outre un système de fermeture et au moins un élément de tension (450) avec une poignée (440), l'au moins un élément de tension (450) s'étendant depuis le système de fermeture et l'au moins un élément de tension (450) est réglable en longueur à partir du système de fermeture par rapport à la poignée (440) et peut être fixé à celui-ci.

5. Orthèse vertébrale (100) de la revendication 4, dans laquelle la poignée (440) définit une poche (460) dans laquelle une première partie d'extrémité de l'élément de tension (450) est retenue.

6. Orthèse vertébrale (100) de la revendication 5, dans laquelle l'élément de tension (450) comporte un dispositif de retenue de câble (456) au niveau de la première partie d'extrémité pouvant être reçu dans la poche (460).

7. Orthèse vertébrale (100) de la revendication 6, dans laquelle la poignée (440) définit une ouverture (452) à travers laquelle l'élément de tension (450) coulisse, le dispositif de retenue (456) empêchant le coulissement de l'élément de tension (450) dans une première direction.

8. Orthèse vertébrale (100) de la revendication 4, dans laquelle la poignée (440) définit une première surface (408) ayant un matériau de crochet agencé pour s'engager avec une surface du premier élément de sangle (114) et une deuxième surface (406) formant une caractéristique de traction.

9. Orthèse vertébrale (100) de l'une quelconque des revendications 1 à 8, dans laquelle la fermeture (400) définit un contour intérieur (426) ayant une forme globalement arquée.

10. Orthèse vertébrale (100) de l'une quelconque des revendications 1 à 9, dans laquelle la fermeture (400) définit des encoches supérieure et inférieure (416).
